(19) Europäisches Patentamt
European Patent Office
Office européen des brevets

(11) Publication number: **0 184 363 A2**

# (12) EUROPEAN PATENT APPLICATION

(21) Application number: **85308534.8**

(22) Date of filing: **25.11.85**

(51) Int. Cl.⁴: **C 12 N 9/96**
A 61 K 37/54, A 61 K 9/22
C 08 G 65/32

(30) Priority: **30.11.84 GB 8430253**

(43) Date of publication of application:
**11.06.86 Bulletin 86/24**

(84) Designated Contracting States:
**BE CH DE FR GB IT LI LU NL SE**

(71) Applicant: **BEECHAM GROUP PLC**
**Beecham House Great West Road**
**Brentford Middlesex TW8 9BD(GB)**

(72) Inventor: **Smith, Richard Anthony Godwin**
**22 Evesham Road**
**Reigate Surrey RH2 9DL(GB)**

(74) Representative: **Valentine, Jill Barbara et al,**
**Beecham Pharmaceuticals Patents & Trade Marks Dept.**
**Great Burgh Yew Tree Bottom Road**
**Epsom Surrey KT18 5XQ(GB)**

(54) **Enzyme conjugates and their therapeutical use.**

(57) A conjugate comprising a plurality of fibrinolytic enzymes linked together through the active centres thereof by means of a removable blocking group.

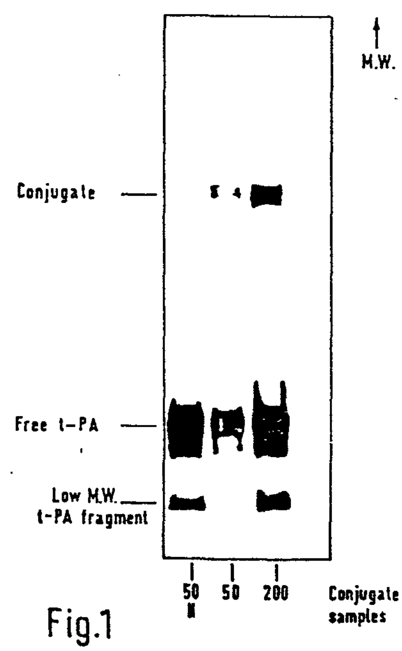


Fig.1

EP 0 184 363 A2

Croydon Printing Company Ltd.

# NOVEL COMPOUNDS

This invention relates to enzyme derivatives, in particular to enzyme derivatives for use in the treatment of thrombotic diseases.

European Patent No.0,009,879 discloses derivatives of in vivo fibrinolytic enzymes which are useful therapeutic agents for treating thrombosis. The derivatives are characterised by the active catalytic site on the enzymes being blocked by a group which is removable by hydrolysis such that the pseudo first order rate constant for hydrolysis is in the range $10^{-6}$ $sec^{-1}$ to $10^{-3}$ $sec^{-1}$.

It has now been found that these enzymes can be linked together by means of a removable blocking group.

According to the present invention there is provided a conjugate comprising a plurality of fibrinolytic enzymes linked together through the active centres thereof by means of a removable blocking group.

The term 'fibrinolytic enzyme' is used herein to mean any enzyme which demonstrates in vivo fibrinolytic activity as defined in European Patent No. 0009 879 (US 4285932). The term thus includes those enzymes which function by direct proteolysis of fibrin (such as plasmin), those which function by activation of plasminogen (such as human tissue plasminogen activator [t-PA], urokinase (both high and low molecular weight forms) and complexes of streptokinase with plasmin). The term also includes those enzymes which can interact with the fibrinolytic system by indirect pathways (such as human activated Protein C). Such enzymes are

obtainable from mammalian urine, blood or tissues or by recombinant DNA methods such as where heterologous host organisms express genes specifying the enzymes. The term also includes:

(a) the fibrinolytically active hybrid proteins as disclosed in European Published Application No. 0155387;

(b) the derivatives of fibrinolytic enzymes in which the catalytic site on the enzyme which is responsible for fibrinolytic activity is blocked by a human protein which is itself a fibrinolytic enzyme, attached thereto by way of a reversible linking group, as disclosed in European Published Application No. 0152736; and

(c) the protein conjugates as disclosed in European Published Application No. 0152736.

The expression ''removable blocking group'' means a group which is reversibly linked to the enzyme such that it is removed under physiological conditions such as human blood.

Suitably the blocking group is removable from each enzyme by hydrolysis at a rate such that the pseudo first order rate constant for hydrolysis is in the range $10^{-6}$ $sec^{-1}$ to $10^{-3}$ $sec^{-1}$ in isotonic aqueous media at pH 7.4 at $37^{0}C$. The preferred rate constants for each link are in the range $1 \times 10^{-5}$ to $8 \times 10^{-4}$ $sec^{-1}$. A suitable medium for measurement of the rate constants is the buffer system consisting of 0.05M sodium phosphate/0.1M sodium chloride/0.01% by volume Tween 80 detergent in water.

Examples of suitable removable blocking groups include groups of structure (I);

$$L\ (-\ B\ -\ A\ -\ X\ -)_n \qquad (I)$$

wherein:

n is an integer of 2 to 6;

L is a nodal group of valency n;

each B is independently a linear hydrophilic linking group;

each A is independently a bridging group comprising at least one heteroatom selected from oxygen, sulphur and nitrogen in which the nitrogen may be optionally substituted by $C_{1-6}$ alkyl; and

each X is independently an acyl group of formula $-\ R\ -\ \overset{O}{\overset{\|}{C}}\ -$ in which R is an aromatic or unsaturated non-aromatic function.

As used herein the expression ''nodal group'' means a polyfunctional linking group.

Preferably n is an integer of from 2 to 4.

Examples of suitable nodal groups L when n is 2 include a bond, a polymethylene group such as methylene or ethylene ($-CH_2-CH_2-$), oxygen, sulphur, dithio (- S - S -), $-S-R^1-$ wherein $R^1$ is derived from a thiol-reactive electrophile; monosubstituted nitrogen i.e imino derivatives such as -NH-; and hydrazine derivatives.

Examples of groups $R^1$ include succinimido and methyleneketo to give groups L:

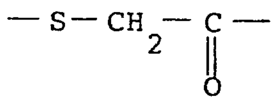

or

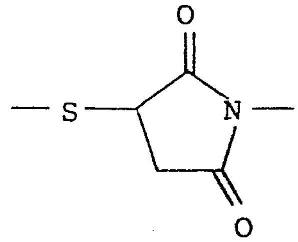

Examples of suitable groups L where n is 3 include unsubstituted nitrogen,

phosphotriester of formula 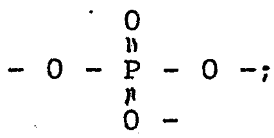;

trihydridic phenoxy such as

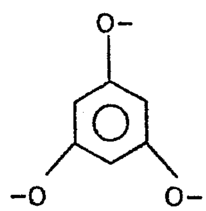

or benzene tricarboxylic acids groups.

Examples of tetravalent nodal groups L include trimethylammonium, carbon and tetrahydric phenoxy units.

Examples of groups B are ω-substituted $C_2$-$C_{10}$ alkanes such as 6-aminohexyl, or linear polymers such as polyethylene glycol or polypropylene glycol optionally terminated by a carbonyl group, poly-glycine, poly-alanine or poly-sarcosine. The linear group may optionally contain a cleavable section such as disulphide bond or α, β dihydroxy function to facilitate analysis of the derivative or to react with the nodal group L.

Preferably B is a linear hydrophilic polymer, for example a polyalkylidene glycol such as polyethylene

glycol or polypropylene glycol optionally terminated by a carbonyl group, or a polyamino acid such as polyalanine and polysarcosine.

It will be appreciated that in groups of structure (I) where n is 2, the moiety $L(-B-)_2$ may therefore itself be a linear hydrophilic polymer such as described above for B.

It will also be appreciated that where the linear hydrophilic polymer is not symmetrical, e.g a polyamino acid, the blocking group as a whole will be assymetric about the nodal group.

In a blocking group of formula (I), preferably all groups B are identical.

Preferably the length of the linear hydrophilic linking groups will be such that the distance between the carbonyl groups of the moieties X attached to the enzymes is at least 30A. In the case where the moiety $L(-B-)_2$ is a polyethylene glycol, the polymer preferably contains 7 to 50 ethyleneoxy units.

Suitable groups A are those which provide sufficient stabilisation of the resulting acyl enzyme to result in a pseudo first order rate constant for hydrolysis in the above mentioned range and preferably in the range 1 x $10^{-5}$ to 8 x $10^{-4}$ $sec^{-1}$.

Examples of A include moieties of formula (II)

$$-(C)_m-D-E \qquad (II)$$

wherein C is a functional group suitable for attachment to B such as $NR^2$, S or O where $R^2$ is hydrogen or $C_{1-6}$ alkyl;

D is a chemical bond or a hydrocarbon chain suitably with up to 8 carbon atoms in the chain;

E is an electron releasing group such as $NR^3$, S or O where $R^3$ is hydrogen or $C_{1-6}$ alkyl; and

m is 1, or when E is suitable for attachment to B, m may instead be O.

Therefore examples of A include;

| | | |
|---|---|---|
| -O- | -S- | $-NH(CH_2)_6NH-$ |
| -NH- | -NH-NH- | $-S(CH_2)_2NH-$ |
| 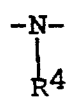 | 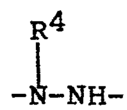 | 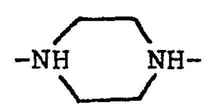 |
| -NH-O- | 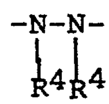 | |
| -O-NH- | $-NH(CH_2)_2NH-$ | |

wherein $R^4$ is a $C_{1-6}$ alkyl group.

Suitable aromatic groups for R include optionally substituted phenyl or naphthyl. Examples of suitable groups X include optionally 2- or 4- substituted benzoyl groups and optionally 2- or 3- substituted acryloyl groups.

Suitable groups X include those groups derived from the blocking groups described in European Patent No. 0,009,879 (US 4285932). Thus, preferred groups are optionally substituted benzoyl or acryloyl groups as described in the above mentioned European Patent, further substituted by the group A, such as optionally substituted benzoyl groups further substituted at the 2 or 4 position by the group A, and optionally substituted acryloyl groups further substituted at the 2 or 3 position by the group A.

The optional substituents for X are suitably electron - releasing substituents such as amino.

Preferred examples of the groups - A -X - are 2- or 4-oxy-, hydrazino-, amino- or ω-aminoalkylamino-benzoic acid derivatives.

Hydrolysis of the above described conjugates under physiological conditions leads to regeneration of the enzyme components in an unmodified form.

The conjugates of the invention may contain more than one type of enzyme. By combination of different enzymes in conjugates of this type, it is possible to manipulate and modify the activities thereof.

Thus, homoconjugates such as dimeric acyl-plasmins or heteroconjugates such as plasmin-t-PA may be prepared. However it is known that the components of the physiological fibrinolytic system interact to different extents and with different sites on the fibrin matrix of a thrombus. It is also known that the physiological clearance rates of the fibrinolytic components differ significantly with, on the one hand, plasminogen being slowly cleared from the circulation and, on the other,

t-PA and u-PA being rapidly cleared. Desirable features of thrombolytic agents include slow physiological clearance and high affinity for fibrin because these properties combine to improve the delivery of proteolytic activity to the thrombus. Therefore, a preferred hetero conjugate is one in which rapidly cleared plasminogen activator is linked to a slowly cleared (acyl-) enzyme (such as [acyl-] plasmin). The resulting conjugate is cleared more slowly than the parent plasminogen activator.

A further preferred heteroconjugate is one wherein affinity for fibrin is conferred upon an enzyme (such as u-PA) which possesses little or no such affinity or is increased by linking enzymes with intrinsic fibrin affinity so as to make possible multivalent interactions with the fibrin matrix.

According to the present invention there is also provided a process for preparing an enzyme conjugate as described above, which comprises reacting one or more fibrinolytic enzymes simultaneously or sequentially with a polyfunctional acylating agent.

Further according to the present invention there is provided a process for preparing an enzyme conjugate as described above which comprises treating an enzyme with a polyfunctional acylating agent.

As used herein, the expression ''polyfunctional acylating agent'' means an agent comprising a removable blocking group such as those of formula (I) above having a plurality of reactive acylating moieties.

In a preferred embodiment conjugates may be prepared by treating one or more fibrinolytic enzymes with an acylating agent of formula (III):-

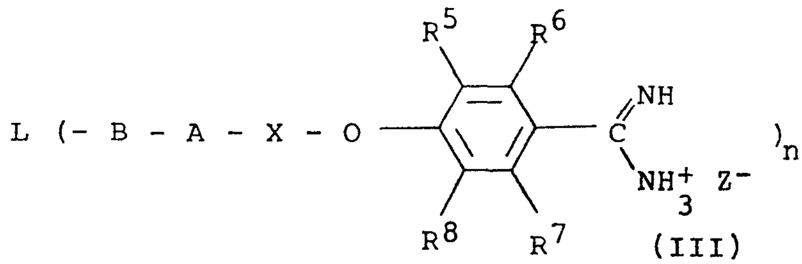

(III)

wherein L, B, A, X and n are defined with reference to formula (I) above; $R^5$, $R^6$, $R^7$, $R^8$, are each independently hydrogen or an electron-withdrawing substituent; and Z is a counter-anion.

Examples of electron withdrawing substituents for $R^5$, $R^6$, $R^7$ or $R^8$ include halogen, nitro or sulphonyl.

Suitable counter-anions Z include chloride, p-toluenesulphonate or trifluoroacetate.

Acylating agents of formula (III) are novel and as such form part of the invention.

If a 'homoconjugate' (containing only 1 type of fibrinolytic enzyme) is required, that enzyme is suitably treated with a $\frac{1}{n} - \frac{1.5}{n}$ - fold molar equivalent of acylating agent of formula (III) where n is as defined in relation to formula (I) above. The treatment is preferably carried out in aqueous buffered medium of preferred pH 6.5-8.5. The enzyme should suitably be at a high concentration, for example at a concentration of greater than 1 mg/ml, preferably from 5-50 mg/ml. The temperature and duration of reaction

are preferably in the range $0^0$-$40^0$C and 30 minutes to 7 days. The acylation of the enzyme may be monitored by measuring the decrease in activity against a convenient substrate (such as a chromogenic tripeptide p-nitroanilide) during coupling. After coupling the conjugate may be purified by standard methods which are discussed below.

Heteroconjugates containing two or more different types of enzyme may suitably be prepared by reacting one enzyme with a 1- to 50-fold molar excess of an acylating agent of formula (III) under the above conditions and then isolating a modified enzyme intermediate in which at least one acylating moiety remains unattached to an enzyme. Suitably, this isolation is carried out by high performance gel permeation chromatography or affinity chromatography. This intermediate is then reacted with the other enzyme(s) under conditions similar to those described above but generally at a lower temperature (preferably $0^0$C to $20^0$C) to minimise deacylation of any acyl-enzyme component.

Optionally, concentration of the enzymes may be combined with coupling by conducting the second step in a centrifugal or pressure-driven ultrafiltration cell and reducing the volume of the reaction mixture during coupling.

Further according to the present invention there is provided a process for preparing an enzyme conjugate as described above, which process comprises reacting together one or more enzymes whose active centres are blocked by groups capable of reacting together to form a reversible blocking group as hereinbefore defined.

In particular, when preparing a conjugate having a blocking group of formula (I) where n=2, the process comprises reacting an enzyme whose active centre is blocked by a group of structure (IV)

$$W - B - A - X - \qquad (IV)$$

with an enzyme whose active centre is blocked by a group of structure (IVA)

$$W^1 - B - A - X - \qquad (IVA)$$

wherein B, A and X are as hereinbefore defined with respect to formula (I) and W and $W^1$ are groups which are capable of reacting together to generate a nodal group L or a precursor thereof.

Examples of suitable groups W and $W^1$ together with the groups L generated thereby can be summarised as follows:

| W | $W^1$ | L |
|---|---|---|
| thio | pyridyldithio | dithio |
| thio | N-maleimido | thio-succinimido |
| thio | halomethylketone (e.g.chloromethylketone) | thio-methylketone |
| hydrazino | aldehyde | substituted hydrazone |

Enzymes blocked by groups of structure (IV) may be prepared by methods analogous to those described in European Patent No 0 009 879 (US 4285932).

The coupling to form the enzyme conjugates may be performed by contacting the enzyme components with an insoluble affinity matrix to which one or both of the components binds. The product(s) may then be isolated by eluting with a suitable ligand. A suitable insoluble affinity matrix for fibrinolytic enzymes is L-lysine-agarose.

The enzyme conjugates obtained may be purified to extract any unreacted starting materials by a variety of techniques. Because the conjugate molecules will generally be of significantly greater molecular weight than the starting enzymes and the polyfunctional acylating agents, size fractionation methods such as gel permeation (and its high performance variant) are particularly useful.

Affinity chromatography methods, particularly those based on the use of L-lysine as a ligand can also be employed.

The acylating agents of formula (III) may be prepared by a variety of standard procedures of which the final step comprises the reaction of a compound of formula (V):

$$L(-B-A-X-OH)_n \qquad (V)$$

where L,B,A,X and n are defined with reference to formula (I) above, with a compound of formula (VI):

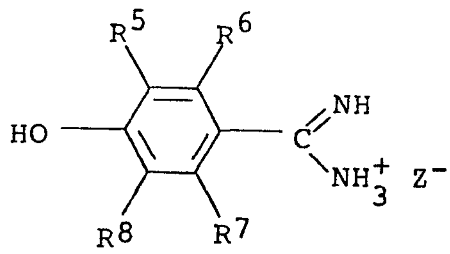

(VI)

where $R^5$, $R^6$, $R^7$, $R^8$ and Z are as defined with reference to formula (III). The intermediate of formula (V) may be prepared by standard procedures.

A preferred general synthetic strategy is as follows:

(a) A compound having the general formula $L(-B-OH)_n$, for example a bi- or polyfunctional polymer (such as polyethylene glycol) is activated by reaction with a molar excess of a reagent such a p-toluenesulphonyl chloride (to give a poly-toluenesulphonate ester) or phosgene (to give a poly-chloroformate). Prior to such activation, a trifunctional polymer may be derived by reacting a trifunctional nodal group precursor such as phosphoryl chloride or benzene (2,4,6) tricarbonyl chloride with an excess of (bifunctional) polyethylene glycol in an anhydrous basic medium such as pyridine. The product may then be isolated from excess unreacted polyethylene glycol and activated as described.

(b) This reactive intermediate is coupled to a nucleophilic derivative of general structure HA - X - OH wherein A and X are as hereinbefore defined in relation to formula (I) under basic

conditions (preferably in the presence of pyridine, triethylamine or aqueous sodium carbonate) at 20-100$^{0}$C for 1-24h.

(c) The intermediate poly-substituted acid from (b) is esterified with a salt of 4-amidinophenol (or a ring-substituted derivative thereof) using a condensing agent such as dicyclohexylcarbodiimide in a weakly basic solvent as described in European Patent No. 0.009,879 (US 4285932) in relation to simple blocking agents. It is preferable to use a slight molar excess of the amidinophenol (1.5 to 4-fold) to ensure efficient esterification. Optionally, the esterification may be performed in the presence of p-toluenesulphonic acid as an acidic catalyst.

The enzyme conjugates of this invention are preferably administered as pharmaceutical compositions.

Accordingly, the present invention also provides a pharmaceutical composition comprising an enzyme conjugate of the invention in combination with a pharmaceutically acceptable carrier.

The composition according to the invention may be formulated in accordance with routine procedures as pharmaceutical compositions adapted for intravenous administration to human beings.

Typically, compositions for intravenous administration are solutions of the sterile derivative in sterile isotonic aqueous buffer. Where necessary the composition may also include a solubilising agent to keep the derivative in solution and a local anaesthetic such as lignocaine to ease pain at the site of

injection. Generally, the derivative will be supplied in unit dosage form for example as a dry powder or water-free concentrate in a hermetically sealed container such as an ampoule or sachette indicating the quantity of enzyme conjugate in activity units, as well as an indication of the time within which the free, unmodified protein will be liberated. Where the derivative is to be administered by infusion, the derivative will be dispensed with an infusion bottle containing sterile pharmaceutical 'water for injection'. Where the derivative is to be administered by injection, the derivative is dispensed by with an ampoule of sterile water for injection. The injectable or infusable composition will be made up by mixing the ingredients prior to administration.

The quantity of material administered will depend on the amount of fibrinolysis required and the speed with which it is required, the seriousness of the thromboembolic condition and position and size of the clot. The precise dose to be employed and mode of administration must <u>per force</u> in view of the nature of the complaint be decided according to the circumstances by the physician supervising treatment. However, in general, a patient being treated for an established thrombus will generally receive a daily dose of from 0.10 to 2.0- mg/kg of body weight either by injection in up to five doses or by infusion. For the treatment of coronary thrombosis a similar dose may be given as a single intravenous bolus.

No toxicological effects have been observed or are indicated at the above described dosage ranges.

Accordingly, in a further aspect of the invention there is provided a method of treating thrombotic disorders, which comprises administering to the sufferer an effective non-toxic amount of a conjugate of the invention.

The invention also provides an enzyme conjugate of the invention for use as an active therapeutic substance and in particular for use in the treatment of thrombotic disorders.

The following Methods and Examples illustrate the invention:

## Methods

### (a) Chromogenic substrate assay

t-PA was assayed against the chromogenic substrate (KabiVitrum, Sweden) S-2288, respectively at a substrate concentration of 1mM in 0.1 M triethanolamine. HCl pH 8.0 at 25$^{o}$C. An SU is defined as the amount of activity that gives an O.D. increase of 0.001/min in 1ml substrate in a 1 cm pathlength cell.

### (b) Rate constant determinations

The pseudo first order rate constant is determined by hydrolysing the acyl-enzyme under physiological conditions, i.e. in isotonic aqueous media at pH 7.4 and at 37$^{o}$C. At regular intervals aliquots are withdrawn and incubated with a chromogenic substrate and the rate of conversion of the substrate measured as indicated above.

The hydrolysis is followed until such time as the rate of conversion of substrate reaches a maximum. The rate constant k is then calculated by plotting:

$$\log_e (1-A^t/A_{max}) \text{ against } t$$

where $A_{max}$ is the maximum rate at which an aliquot converts substrate and $A_t$ is the rate at which an aliquot converts substrate at time t.

EXAMPLE 1

α,ω-O,O'-bis-(4-carboxyphenyl)polyethylene glycol 400, bis-4-amidinophenyl ester p-toluenesulphonate

(α,ω bis-(4-carboxyphenyloxy)polyethylene glycol 400, bis-4-amidinophenyl ester. p-toluenesulphonate)

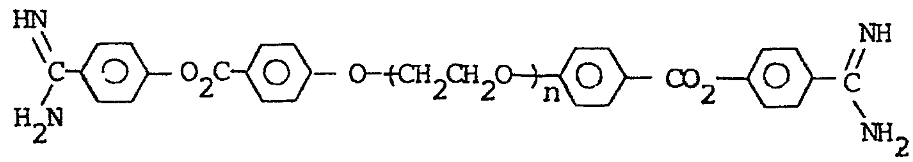

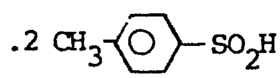

n ~ 8 or 30

Polyethylene glycol 400 (3.70 g) was mixed with p-toluenesulphonyl chloride (4.19 g) in dry pyridine (5.0 ml), allowed to cool at 4°C for 70h and poured into crushed ice (~ 50 ml). The aqueous layer was extracted with methylene chloride (100 ml) and the organic layer washed twice with 6 N HCl saturated with NaCl and then dried. The organic layer was evaporated to dryness at 35°-40°C and the whole of the solid dissolved in tetrahydrofuran (50 ml) containing potassium hydroxide (1.45 g), p-hydroxybenzoic acid methyl ester (3.04 g) and water (1.0 ml). The mixture was heated (70°C) for 95h, cooled, and water (40 ml), sodium hydroxide (0.2 g), methanol (16 ml) and methylene chloride (50 ml) added. The organic layer was separated, washed with saturated brine and dried over anhydrous sodium sulphate.

The product was hydrolysed to the acid by refluxing for 4h in methanol (50 ml) containing KOH (0.15 g), pouring into water (300 ml), acidifying to pH 1.0 with concentrated HCl and extracting with chloroform (100 ml). After drying and evaporation, 3.06 g (50%) of an oil which gave a solid acid were obtained. The $^{1}H$ nmr spectrum of this material was consistent with the structure of the intermediate acid. This intermediate (0.61 g) was dissolved in thionyl chloride (5 ml) at ambient temperature for 30 min and excess thionyl chloride removed azeotropically by evaporation from 2 volumes of benzene. The intermediate acid chloride was dried *in vacuo* for 15 min and then added to pyridine (5 ml) and p-amidinophenol hydrochloride (1.73 g). The mixture was stirred at ambient temperature for 70h. Solvent pyridine was removed by evaporation *in vacuo* and the residue triturated with diethyl ether (100 ml). Isopropanol (25 ml) was added and the product dissolved on heating, depositing an oil on recooling. This oil was mixed with p-toluenesulphonic acid (0.69 g) and recrystallised from isopropanol to yield a small quantity (c. 100 mg) of a white solid. m.p. $147^{o}$-$148^{o}C$.
The material was characterised by $^{1}H$ nmr which showed that it contained some monoester as well as diester. Further purification could be effected using a column (40 ml) of Sephadex® LH20 eluted with neat methanol.

The analogue with about 30 ethylenoxy units in the bridge was prepared in a similar fashion starting from polyethylene glycol 1500.

EXAMPLE 2

α,ω-O,O'-bis-[N-2-carbonyl(4'-hydrazinobenzoic acid)] polyethylene glycol 600, bis-4-amidinophenyl ester hydrochloride

(α,ω bis- [N-2-carbamyl (4'-hydrazinobenzoic acid)] polyethylene glycol 600, bis-4-amidinophenyl ester hydrochloride)

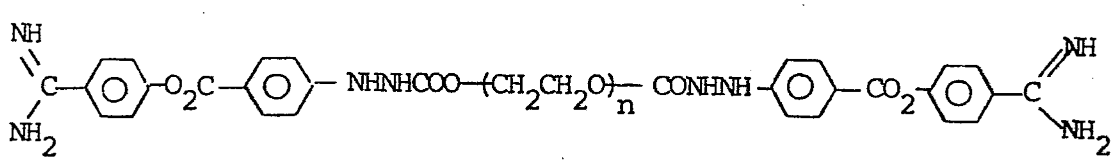

.2HCl

n ~13

Polyethylene glycol 600 (6.0 g) was dissolved in a 12.5% w/v solution of phosgene in toluene (80 ml) and stirred at ambient temperature for 3 days. Excess phosgene and toluene were removed by evaporation and the residual oil dissolved in dry pyridine (40 ml). 4-hydrazinobenzoic acid (3.04 g) was added and the mixture stirred for 16h at ambient temperature. The product was precipitated with 2 volumes of dry diethyl ether, redissolved in pyridine (50 ml) and re-precipitated with ether. The precipitated oil was dissolved in dry pyridine (50 ml) and p-amidinophenol hydrochloride (5.16 g, 3-fold molar excess), dicyclohexyl carbodiimide (4.53 g) and anhydrous p-toluene sulphonic acid (50 mg) added. The mixture was stirred

for 2 days at ambient temperature, filtered and the filtrate precipitated with dry diethyl ether (2 volumes). The resulting gum was recrystallised from: (1) 2-propanol (200 ml) and diethyl ether (20 ml); (2) ethanol (50 ml) and diethyl ether (30 ml). The product was formed as a gum on cooling at 4°C and was washed with diethyl ether and then dried in vacuo over $P_2O_5$. A pale orange amorphous solid resulted (2.1 g, 20%). m.p. 46°-48°C.

$^1$H nmr (DMSO $d^6$) δ, 9.25/9.45 (broad d, 8H exchangeable with $D_2O$, amidine-H), 7.95/7.50 (dd, 8H, amidinophenyl H), 7.95/6.78 (dd, 8H, hydrazinobenzoyl H), 4.16 (broad S, 4H, methyleneoxycarbonyl H), 3.65-3.35 (envelope, 56H, alkoxy backbone H)

EXAMPLE 3

α,ω-O,O'-bis-[N-6-(N'-carbonylamino)hexylanthranilic acid]polyethylene glycol 600, bis-4-amidinophenyl ester hydrochloride.

(α,ω bis-[N-6-carbamylhexylanthranilic acid] polyethylene glycol 600, bis-4-amidinophenyl ester hydrochloride)

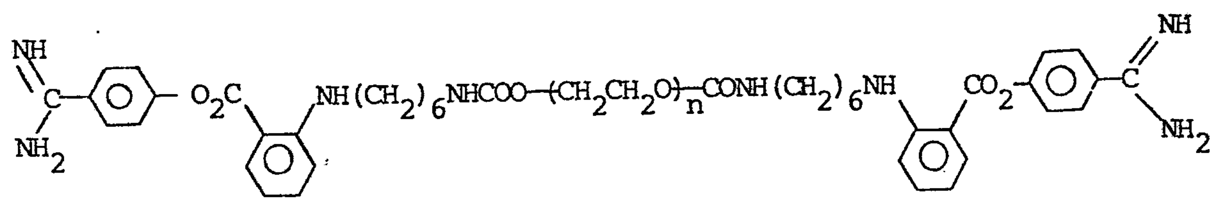

.2HCl n ~13

Polyethylene glycol 600 (3.0 g) was dissolved in 12.5% w/v solution of phosgene in toluene (80 ml) and stirred at ambient temperature for 16h. Excess phosgene and toluene were removed by evaporation and the residual oil added to a suspension of N-6-(aminohexyl)anthranilic acid in 1.5 M sodium carbonate solution (20 ml). An oil was formed and the mixture was stirred at ambient temperature for 6h. The product was evaporated to dryness and extracted with methanol (70 ml) at 40°C. The methanol extract was evaporated and the diacid intermediate dried *in vacuo* over $P_2O_5$. The whole of the resulting glassy solid was dissolved in dry pyridine (40 ml) containing dicyclohexyl carbodiimide

(4.12 g), p-amidinophenol hydrochloride (6.9 g) and p-toluenesulphonic acid monohydrate (1.9 g). The mixture was stirred at ambient temperature for 2 days, filtered and the filtrate poured into dry diethyl ether (200 ml) at 4°C. After 3 days at 4°C, a gummy solid was deposited, the supernatant was decanted off and the residue was dissolved in hot isopropanol (100 ml). Diethyl ether was added to the cloud point (20 ml) and a yellow oil was deposited over 7 days at 4°C. The process was repeated twice and the final product dried *in vacuo* to give a gum (total recovered weight: 1.74 g).

$^1H$ nmr (DMSO $d^6$) δ; 9.25/9.50 (d, 4H exchangeable with $D_2O$: amidine), 6.5-8.2 (m, 8H, aromatic protons); 3.8-4.2, 2.7-3.7, 0.9-1.8 alphatic protons. Integration indicated a diester content of about 70%.

EXAMPLE 4

α,ω-O,O'-bis-(N-[2-(N'-carbonylaminoethyl)-4'-amino] benzoic acid)polyethylene glycol 600, bis-4-amidinophenyl ester hydrochloride.

(α,ω bis- [N-2-carbamyl {4-(2-aminoethylamino)benzoic acid}] polyethylene glycol 600, bis-4-amidinophenyl ester hydrochloride)

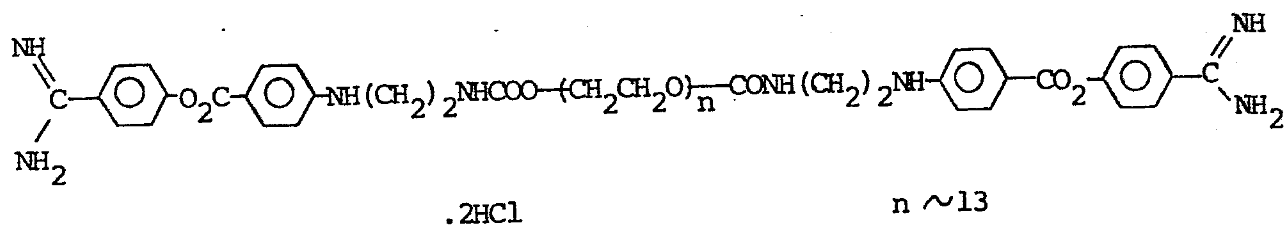

.2HCl n ~13

This compound was prepared in six steps.

1. Benzyl 4-aminobenzoate

4-Aminobenzoic acid (5.0 g) was suspended in thionyl chloride (50 ml) and the mixture heated to reflux under nitrogen, until a clear yellow solution had formed. The thionyl chloride was removed by evaporation, the last traces by azeotrope with dichloromethane (3 x 50 ml). The solid 4-sulphinylaminobenzoyl chloride (IR 1780, 1745 $cm^{-1}$) was dissolved in dichloromethane (50 ml) and cooled in an ice-bath. To the stirred reaction mixture was added a solution of benzoyl alcohol (12 ml) in dichloromethane (50 ml). After stirring overnight the white solid was removed by filtration and the solid was washed with dichloromethane (50 ml). The solid was suspended in, and triturated with 10% sodium bicarbonate solution (50 ml). This was decanted off, and another aliquot (50 ml) of the bicarbonate solution introduced. The suspension was stirred for 1h, and the mixture transferred to a separating funnel, and extracted with dichloromethane (3 x 150 ml). The organic layer was dried (sodium sulphate), filtered and evaporated to leave a yellow solid (5.61 g).

$^{1}H$ nmr ($CDCl_3$) δ, 7.80 (d, 2H, J = 9 Hz), 7.30 (m, 5H, aryl-H), 6.45 (d, 2H, J = 9 Hz, aryl-H), 5.25 (S, 2H, Aryl-$CH_2$), and 4.0 (S, 2H, $NH_2$).

Infra red: (Nujol) 3450, 3350, 3250, 1680, 1630, 1600, 1510, 1275, 1170, 1110, 840, 770, and 690 $cm^{-1}$.

2. Benzyl 4-N-(2-phthalimidoethyl)aminobenzoate

Benzyl 4-aminobenzoate (2.92 g) and 2-bromoethylphthalimide were heated to 100°C under an atmosphere of nitrogen to produce an orange oil. TLC analysis ($SiO_2/CH_2Cl_2$) after 36h showed the appearance of a product spot, and also much polymeric material. Chloroform soluble material was isolated by trituration and filtration, and the chloroform extract was evaporated. Column chromatography (50 g silica, dichloromethane), led to isolation of an oil, which was further purified by recrystallisation (chloroform/ 40-60° petroleum ether) to leave a pale yellow crystalline material (410 mg).

$^1H$ nmr ($CDCl_3$) δ, 8.0 (M, 6H, aryl-H), 7.25 (S, 5H, aryl-H), 6.50 (d, 2H, J = 9 Hz), 5.25 (S, 2H, aryl-$CH_2$), 4.5 (Irr S, 1H, N-H), 3.90 (t, 2H, J = 6 Hz, $CH_2HCO$), and 3.40 (t, 2H, J = 6 Hz, $CH_2NH$).

Infra Red: (Nujol) 3480, 1775, 1705, 1610, 1530, 1280, 1175 and 715 $cm^{-1}$.

m.p. 118-9°C.

Anal. found C 68.37, H 4.98, and N 6.44%. $C_{24}H_{20}N_2O_4 \cdot H_2O$ requires C 68.85, H 5.30 and N 6.69%.

3. Benzyl 4-N-(2-aminoethyl)aminobenzoate

The phthalimide (410 mg) was dissolved in ethanol (10 ml) and 100% hydrazine hydrate (60 µl) was added. This was stirred at reflux for 18h, and concentrated hydrochloric acid (1.0 ml) was added to the hot solution. Reflux was continued for 0.5h further and the solution was allowed to cool. The precipitate was isolated by filtration and the solid washed with water (5.0 ml). The ethanol was removed by evaporation at reduced pressure to leave a mainly aqueous solution which was refiltered. The aqueous layer was basified with 2 M sodium hydroxide solution, and was extracted with ethyl acetate (3 x 25 ml). The organic layer was dried (sodium sulphate), filtered and evaporated to leave an oil (229 mg, 83%).

$^1$H nmr ($CDCl_3$) δ; 7.8 (d, 2H, J = 8 Hz, aryl-H), 7.2 (S, 5H, Ph-H), 6.45 (d, 2H, J = 8 Hz, aryl-M), 5.20 (S, 2H, aryl-$CH_2$), 4.5 (br S, 1H, N-H), 3.2 (br S, 4H, $CH_2CH_2$), and 1.8 (S, 2H, br S, $NH_2$).

Infra Red: (thin film) 3370, 1690, 1600, 1520, 1170, 1100, 910, 840, 770 and 690 $cm^{-1}$.

4. α,ω-O,O′ -bis-(N-[2-(N′-(carbonylaminoethyl)-4′-amino] benzoic acid) polyethylene glycol 600, bis-benzyl ester

Polyethyleneglycol 600 (82 mg) was dissolved in toluene (5 ml). A 12.5% solution of phosgene in toluene (260 µl) was added to the ice-cooled mixture. After stirring for 1h, all volatile material was removed and toluene (5 ml) was added to the oil. A solution of benzyl N-(2-aminoethyl)-4-aminobenzoate (73 mg) and triethylamine (50 µl) in toluene (5 ml) was added. The mixture was stirred overnight and the toluene layer was washed with a saturated solution of sodium sulphate (2 ml). The organic solution was dried (sodium sulphate), filtered and evaporated to leave an oil (120 mg, 72%).

$^1$H nmr ($CDCl_3$) δ; 7.75 (d, 4H, J = 9 Hz, aryl-H), 7.30 (S, 10H, aryl-H), 6.50 (d, 4H, aryl-H), 5.5 (br S, 2H, NHCO), 5.25 (S, 4H, $CH_2Ph$), 4.90 (br S, 2H, NHAr), 4.20 (M, 4H, O=C-O-$CH_2$), 3.55 (S, 50H, PEG), and 3.3 (br S, 8H, $NHCH_2$).

Infra Red: (neat) 3350, 1710, 1605, 1495, 1280, 1100, 1020, 730 and 695 $cm^{-1}$.

5. α,ω-O,O′-bis-(N-[2-(N′-carbonylaminoethyl)-4′-amino] benzoic acid) polyethylene glycol 600

The benzyl ester (120 mg) was dissolved in methanol (10 ml) and 10% Pd on charcoal "catalyst" was added. The mixture was hydrogenated until hydrogen uptake stopped. The mixture was filtered through a pad of celite, and the solution was evaporated to leave an oil (70 mg).

$^1$H nmr ($CDCl_3$/$d^6$-acetone) δ; 7.8 (d, 4H, J = 8 Hz, aryl-H), 5.3-6.8 (d, 10H, on broad hump aryl-H and acidic-H), 4.2 (br S, 4H, $OCH_2$), 3.6 (S, 50H, PEG-H), and 3.4 (br S, 5H, $NHCH_2$).

Infra Red: (neat) 2400-3600, 1710, 1605, 1530, 1260, 1010, 775 and 670 $cm^{-1}$.

6. Title compound

The PEG-diacid (70 mg, 67 μmole) was dissolved in pyridine (1 ml) and 4-amidinophenol (46 mg, 270 μmole) was added. This was followed by dichyclohexylcarbodiimide (28 mg, 135 μmole) and 4-toluene sulphonic acid (2 mg). The mixture was stirred for 5 days and the solid formed was removed by filtration through a plug of glass wool. The solution was evaporated to leave an oil (138 mg).

EXAMPLE 5

α,ω-O,O'-bis-(N-[2-(N'-carbonylaminoethyl)-4'-amino] benzoic acid)polyethylene glycol 600, bis-2-chloro-4-amidinophenyl ester hydrochloride.

(α,ω bis-[N-2-carbamyl {4-(2-aminoethylamino)benzoic acid}] polyethylene glycol 600 bis 2-chloro 4-amidinophenyl ester hydrochloride)

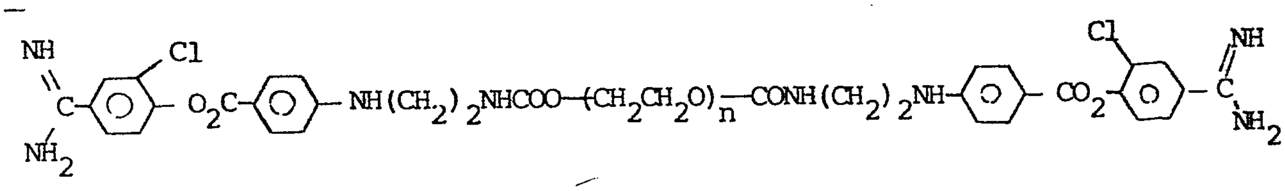

.2HCl n ∼13

The PEG-diacid from Example 4 step 5 (38 mg, 36.3 μmole) and 2-chloro-4-amidinophenol (15 mg, 76 μmole) were dissolved in pyridine (0.5 ml). Dicyclohexylcarbodiimide (15 mg, 76 μmole) was added, followed by dry 4-toluene sulphonic acid (1 mg). The mixture was stirred for 6 days in which time a white precipitate had formed. The solution was filtered through a pad of glass wool and evaporated to dryness, leaving a gummy oil (50 mg).

EXAMPLE 6

α,ω-O,O'-bis-(4-carboxyphenyl)polyethylene glycol 400, bis-[human $lys_{77}$-(ser 740-yl)plasmin]ester

(α,ω bis-(4-carboxyphenyloxy) polyethylene glycol 400, bis-[human $lys_{77}$-(ser 740-yl) plasmin] ester)

Human plasmin was obtained by urokinase activation of human $lys_{77}$ plasminogen (KabiVitrum, Sweden) and was used at a concentration of 837 µM (determined by active site titration, Chase J and Shaw E. Biophys. Res. Commun. 1967 29: 508-514) in 0.1 M trishydroxymethylaminomethane.HCl, 0.9% w/v NaCl, 20% v/v glycerol [TGS buffer]. 200 µl of this solution was mixed with the bifunctional ester of Example 1 (8 µl of a 10 mM solution in dimethylsulphoxide) and incubated at $0^{O}C$ overnight. 100 µl of this mixture was mixed with 0.08 M sodium phosphate, 0.32 M NaCl, 20% v/v ethanol buffer, pH 7.0 [PSE buffer, 100 µl] and purified by high performance gel permeation chromatography under the following conditions:

Column: TSK modified silica gel permeation column G 3000 SW (600 x 7.5 mM) calibrated with protein molecular weight standards (Biorad, U.K.)

Buffer: PSE

Flow rate: 0.5 ml/min

The effluent absorbance at 280 was monitored and two peaks were detected at eluted volumes of 13.1 and 17.7 ml corresponding to molecular weights of about 160 and 80 kilodaltons (dimeric and monomeric plasmin respectively).

No significant amount of higher oligomer was observed indicating specific dimerisation. Fractions containing the first peak were pooled (3.0 ml) and saturated ammonium sulphate solution (6.0 ml) added. The mixture was held on ice for 1 hour and centrifuged at 1500 g for 30 min at 4$^{o}$C. The precipitated protein pellet was stored at -40$^{o}$C and then dissolved in the above TGS buffer (1.0 ml) containing 0.01% w/v Tween 80 detergent. This solution was incubated at 37$^{o}$C and assayed using the plasmin-specific substrate S-2251 (see Methods). The initial amidolytic activity was 1.3% of that attained after 6 hours incubation, indicating that the product was isolated in an initially inactive but regeneratable form. Analysis of the deacylation kinetics (see methods) gave an apparent pseudo first order rate constant for deacylation of 1.2 x $10^{-4}$ $sec^{-1}$ at pH 7.4, 37$^{o}$C.

EXAMPLE 7

α-[human(ser 478-yl)tissue plasminogen activator], ω-[human lys$_{77}$-(ser 740-yl)plasmin], O,O'-bis-(N-2-carbonyl[4'-hydrazinobenzoic acid])polyethylene glycol 600.

(α-[human (ser 478-yl) tissue plasminogen activator], ω-[human lys$_{77}$-(ser 740-yl) plasmin), bis-N-2-(carbamyl {4'-hydrazinobenzoic acid}) polyethylene glycol 600)

This compound was prepared in two steps.

(1) α-[4-amidinophenyl], ω-[human lys$_{77}$-(ser 740-yl) plasmin] bis-N-2 (carbamyl {4'-hydrazinobenzoic acid}) polyethylene glycol 600

Human lys$_{77}$-plasmin (1.6 ml of a 430 μM solution) was mixed with a solution of the bifunctional ester of Example 2 (0.4 ml of 20 mM in DMSO, 11-fold molar excess over plasmin) and incubated for 1h at ambient temperature and then at 0$^{o}$C for 1h. The product was fractionated by high performance gel permeation chromatography under conditions similar to those used for Example 6 above except that a preparative-scale column of 300 x 22.5 mM and a flow rate of 1.0 ml/min were used. A small peak corresponding to the dimeric plasmin analogue of Example 6 was observed together with a much larger peak of c. 80 kilodaltons well separated

from the lower molecular weight acylating agent. Fractions eluting at 59-69 ml (80-90 kilodaltons) were collected, pooled and exchanged into 1% w/v D-mannitol, 20 mM ammonium bicarbonate, 1.0 mM 6-aminohexanoic acid pH 7.4 (MAE buffer) using small columns of Sephadex (TM) G-25 M (Pharmacia, Sweden). The column eluate was pooled and lyophilised to yield a white powder (199.7 mg).

(2) Title Compound

Human Tissue plasminogen activator was purified from the culture supernatant of Bowes melanoma cells (Rijken D,L, Collen D. J. Biol. Chem. 1981 256:7035-7041) and radiolabelled with Na $^{125}I$/iodogen (Fraker P J, Speck J C. Biochem. Biophys. Res. Commun. 1978 80:849-857) The activator ( 1 nanomole, 0.5 µCi) in 0.05 M sodium phosphate 0.145 M NaCl pH 7.4 (PBS buffer, 1.0ml) was mixed with the above plasmin monoester (68.2 mg) and bovine lung protease inhibitor (aprotinin, 44 nanomoles). The mixture was placed in a Centricon (TM) centrifugal ultrafiltration cell (Amicon, U.K., 10,000 dalton MW cut-off membrane) and concentrated at 5000 g/4°C for 3h. At the end of this period the volume of the reaction mixture was 0.19 ml and the total amidolytic activity of the t-PA had decreased to 13.3% of the original value. The coupled product was stored in frozen solution at -196°C and an aliquot (80 µl) was diluted into TGS buffer (420 µl) containing 18 µM aprotinin. This solution was deacylated at 37°C and the amidolytic activity measured using the t-PA-specific substrate S-2288

(see Methods) in the presence of aprotinin (4.5 μM). t-PA amidolytic activity was regenerated with a pseudo first order rate constant of $2.91 \times 10^{-4}$ $sec^{-1}$. When the unhydrolysed product was chromatographed using high performance gel chromatography under the conditions described in Section (1) above, major peaks of radioactivity were found at eluate volumes of approximately 60 and 73 ml. These correspond

to species with molecular weights of about 145 and 60 kilodaltons respectively. Material from the high molecular weight peak was subjected to polyacrylamide gel electrophoresis in sodium dodecyl sulphate followed by fibrin overlay zymography (Granelli-Piperno A, Reich E. 1978 Journal of Experimental Medicine. 148:223-234). The results are shown in Fig. 1. The conjugate showed a band at about 150 Kilodaltons which disappeared after hydrolysis in TGS buffer at 37°C for 4h. Bands corresponding to free tissue plasminogen activator (63 kilodaltons) and plasmin (~90 kilodaltons) appeared on hydrolysis.

EXAMPLE 8

α-[human(ser 478-yl)tissue plasminogen activator], ω-[human lys77-(ser 740-yl)plasmin], O,O'-bis-(N-[2-(N'- carbonylaminoethyl)-4'-amino] benzoic acid)polyethylene glycol 600.

(α-[human (ser 478-yl) tissue plasminogen activator], ω-[human $lys_{77}$-(ser 740-yl) plasmin, bis-[N-2-carbamyl {4-2-aminoethylamino)benzoic acid}] polyethylene glycol 600)

This compound was prepared in two steps.

(1) α-[4-amidinophenyl], ω-[human $lys_{77}$-(ser 740-yl) plasmin, O,O'bis-(N-[2-(N'-2-carbonylaminoethylamino)benzoic acid)] polyethylene glycol 600

Human $lys_{77}$-plasmin (1.8 ml of 430 μM solution) was mixed with a solution of the bifunctional ester of Example 4 (200 μl of 20 mM in dimethylsulphoxide) and incubated at 37°C for 1h. After this time 5.7% of the original amidolytic activity of the plasmin remained. The product was purified as described for the intermediate of Example 7 (1) and yielded a by-product corresponding to the dimeric acyl plasmin (elution volume 57 ml) and a main product eluting at 63-68 ml. The latter was exchanged into MAE buffer diluted 1:1 with water and lyophilised. A white powder

(60.2mg) resulted. On reconstitution in TGS buffer containing 0.01% w/v Tween 80 and incubation at 37$^{o}$C, this acyl-enzyme deacylated with a pseudo first order rate constant of 1.2 x $10^{-4}$ $sec^{-1}$.

(2) Title compound

The above plasmin monoester (32.4 g) was mixed with radiolabelled human tissue plasminogen activator (approx. 8 nanomoles, 0.86 μCi) in PBS buffer (1.0 ml) containing 22 μM aprotinin. The mixture was placed in a Centricon(TM) vial with a 30,000 dalton molecular weight cut-off and centrifuged at 5000 g for 3.5h at 10$^{o}$C. The solution was concentrated to about 50 μl. The product was purified by high performance gel permeation chromatography as described for Example 7 (1) except that a flow rate of 0.75 ml/min was used. Two peaks of radioactivity were observed, the first at about 60-65 ml and the second at 70-80 ml. The high molecular weight fraction was subjected to SDS-PAGE/fibrin overlay zymography and this showed (Fig. 2) a fibrinolytic component at high molecular weight which was convertable to free t-PA on hydrolysis.

Example 9

α,ω-0,0′-Bis - [N-2-carbonyl-(4'-hydrazinobenzoic acid)]polyethylene glycol 1500, bis 4-amidinophenyl ester hydrochloride

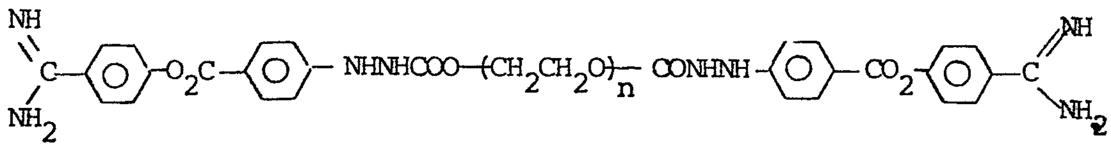

.2HCl n ~ 30

This material was prepared in the same way as that described for Example 2 except that polyethylene glycol 1500 was used and a 4-fold molar excess of p-amidino phenol was employed at the esterification stage. The product was purified by recrystallisation three times from ethanol/diethyl ester and was isolated as an orange gum. The $^1H$ NMR spectrum of this material was very similar to that of the compound of Example 2 with the exception that the ratio of high-field signals corresponding to the polyethylene glycol backbone (δ: 3.3-3.7) to those corresponding to the aromatic region (δ: 7.4-8.0) was higher, reflecting the longer polyethylene glycol bridge.

Example 10

α,ω -bis- (human [ser 478-yl]-tissue plasminogen activator), 0,0′-bis-(N-2-carbonyl-[4'-hydrazinobenzoic acid]) polythene glycol 600.

Recombinant tissue-type plasminogen activator (131.2 nmol) was precipitated from a stock solution of 43.7μM

in 0.5M L-arginine, 0.5M NaCl, 20mM trishydroxymethylaminomethane, HCl, 0.01% w/v Tween 80 pH 7.4 (ANT Buffer) using saturated ammonium sulphate solution and centrifuging at 7,500g for 30 mins at 4°C. The precipitate was suspended in 0.3ml ANT buffer (final vol 0.5ml) and assayed at 243µm. The compound of Example 2 (9.1µl of 10mM in DMSO, 0.75 equivalents) was added and the mixture incubated at 0°C. After a total of 6h at 0°C, the mixture contained 7% of the original amidolytic activity against S-2288 substrate and was diluted into ANT buffer (1.5ml). The product was chromatographed under the conditions described for Example 7(1) except that ANT buffer was employed at a flow rate of 2.0ml/min and a preparative pre-column was used. A peak at 92ml corresponding to a molecular weight of 130 kilodaltons was collected (18ml) and precipitated with saturated ammonium sulphate (36ml) at 17,500g, 4°C for 60 min. The product was dissolved in ANT buffer (1.50ml) and stored at -70°C. In TGS buffer at 37°C, the product deacylated with an apparent first order rate constant of $4.48 \times 10^{-4}$ $sec^{-1}$

Example 11

Bis-α,ω-(Human [Ser 478-yl] tissue plasminogen activator)-O,O′-bis-(N-2-carbonyl-[4′-hydrazinobenzoic acid])polyethylene glycol 1500

Human recombinant tissue-type plasminogen activator (80.2 nmol) was dialysed into 2.6ml of 0.05M sodium phosphate 0.1M sodium chloride, 0.01% w/v Tween 80 [PST buffer] and 8µl of the compound of Example 9 (5mM in DMSO) added [0.5 equivalents]. After 2h at 0°C, a further 8µl of acylating agent was added and incubation continued at 0°C for 16h. Saturated ammonium sulphate

solution (5.2ml) was added to the product and the precipitate isolated by centrifugation at 3000g, 4°C for 15 min. The precipitate was dissolved in ANT buffer (0.5ml) and PSE buffer containing 0.1M 6-aminohexanoic acid [PSEE] (0.5ml) and filtered. It was chromatographed as described in Example 6 but using PSEE buffer and a flow rate of 0.2ml/min. Two peaks were observed eluting at 19 and 22.2ml. The former was isolated by gel filtration (Sephadex G-25) into ANT buffer (3.0ml) of the pooled eluate at 18-20ml. The product was stored in solution at -70°C and had an apparent first order deacylation rate constant in PST buffer at 37°C of 2.3 x $10^{-4}$ $sec^{-1}$. When subjected to gel electrophoresis and fibrin overlay zymography as described in Example 7, the product showed a band at 130 kilodaltons which was sensitive to hydrolysis.

Example 12

α-[Human (Ser 478-yl) tissue plasminogen activator], ω-[Human $lys_{77}$-(Ser 740-yl) plasmin],0,0′-bis-(N-2-carbonyl-[4′-hydrazino benzoic acid])polyethylene glycol 1500

This compound was prepared without isolation of the intermediate acyl-plasmin.

Human plasmin (1.5ml of 286µm in TGS buffer) was treated with the compound of Example 9 (103µl of 5mm in DMSO, 1.2 equivalents) for 2h at 0°C. The amidolytic activity of the plasmin was reduced by 97%. The product was mixed with aprotinin (222 nmoles) and gel filtered into PST buffer containing 10mM 6-aminohexanoic acid and 20µM aprotinin (3.0ml) at 4°C using small Sephadex G-25 columns as described above. Recombinant tissue type plasminogen activator in ANT

buffer (4.0ml, 233 nmoles) was precipitated with saturated ammonium sulphate solution (12ml) at 10,000g 14°C for 1h. The pellet was dissolved in the above acyl-plasmin solution and an additional 222 nmoles of aprotinin added. The product was reduced to 2.3ml by centrifugal concentration as described in Example 7 over 4½ at 4°C and frozen at -70°C. After storage at this temperature for 72h and thawing, the mixture retained 37.5% of the original t-PA activity against substrate S-2288. The product was fractionated using preparative high performance gel filtration under the conditions described in Example 10 but with a flow rate of 1.5ml/min. The product eluting between 75 and 96ml was pooled and precipitated with saturated ammonium sulphate (24ml) at 10,000g, 4°C, 1h. The precipitate was dissolved in ANT buffer (1.0ml) and stored at -70°C.

The product deacylated with an apparent first order deacylation rate constant of $2.06 \times 10^{-4}$ $s^{-1}$ in PST buffer at 37°C. Gel electrophoresis and fibrin overlay zymography (as described in Example 7) showed a fibrinolytic band at 143 kilodaltons which was susceptible to hydrolysis.

Example 13

α-[Human (Ser 478-yl) tissue plasminogen activator], ω-[Human $lys_{77}$-(Ser 740-yl)plasmin], 0,0′-bis-N-6-[N′-carbonylamino]hexylanthranilic acid polyethylene glycol 600.

This compound was prepared in two steps.

(1) α-[4-amidinophenyl],ω-[human $lys_{77}$-(Ser 740-yl)-plasmin], 0,0′-bis-N-6-[N′-carbonylamino]-hexylanthranilic acid polyethylene glycol 600.

Human $lys_{77}$-plasmin (1.9ml of a 430µm solution) was mixed with the acylating agent of Example 3 (80 µl of 50mM) and incubated initially for 150 min at 25°C, then at 40°C overnight and then, after addition of a further 80µl of acylating agent at 37°C for 75 min. Approximately 94% inactivation of the plasmin occurred. Remaining plasmin was neutralised by addition of aprotinin (80 nmoles) and chromatographed as described in Example 7(1) except that a flow rate of 1.5ml/min was used. The protein eluting between 62-72ml was collected and gel filtered into MAE buffer as described in Example 7(1). The column eluate was lyophilised to yield a white powder (149.5 mg). The product was approximately 10% protein by weight and deacylated in TGS buffer at 37°C with a pseudo first order rate constant of $5.46 \times 10^{-5} sec^{-1}$.

(2) α-[Human (Ser 478-yl) tissue plasminogen activator],ω-[Human $lys_{77}$-(Ser 740-yl)plasmin], O,O′-bis-N-6- [N′-carbonylamino]hexylanthranilic acid polyethylene glycol 600.

The above compound (64.3mg) was mixed with PBS buffer (0.9ml), aprotinin (80 nmol, 0.1ml) and human t-PA (80 nmol, containing $^{125}$ I-labelled t-PA as described in Example 7 (2) above) and concentrated by centrifugation as described in Example 7 (2). The final volume was 0.3ml and the t-PA was approximately 70% inactivated. The product was purified by high performance gel chromatography as described in Example 7 (2). The radioactive protein eluting at 56-64 ml was pooled, gel filtered into MAE buffer containing 1mg/ml human serum albumin as described above, and lyophilised to yield 123 mg of a white solid. This material deacylated at 37°C with an apparent first order rate constant of $1.0 \times 10^{-4} sec^{-1}$ in TGS buffer.

Example 14

N,N-(6,6)-[(3,3-dithiobispropionyl)-N-(6-aminohexyl)-anthranilic acid]-bis-(2-chloro-4-amidino)phenyl ester hydrochloride

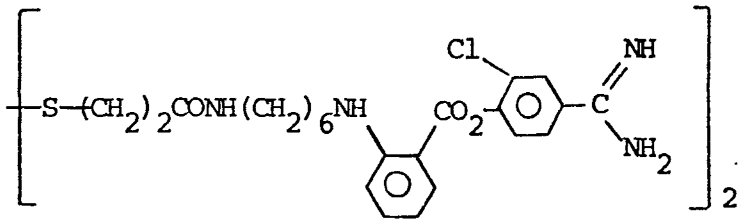

.2HCl

(1) N,N-(6,6)-[(3,3-dithiobispropionyl)-N-(6-aminohexyl)anthranilic acid].

3,3-dithiopropionic acid bis-N-oxysuccinimide ester (1.0g) was added to a suspension of N-6-aminohexylanthranilic acid (1.25g, 0.5mol equivalent) in dry pyridine (10 ml). The mixture was stirred for 2 days at ambient temperature and evaporated to an oil which was dissolved in 2N HCl (40 ml) and then adjusted to pH 2.0 with 5N NaOH. A white solid precipitated on standing at 40°C and was recrystallised from ethanol/water 1:1 v/v (50 ml), and ethanol/water/acetone 1:2:2 v/v (40 ml). An off-white solid resulted which was dried in vacuo to yield 0.9 g of product m.p 133 - 135°C.

'H Nmr (DMSO $d^6$) δ; 7.8 (irregular d, 1H, $H_6$): 7.35 (t, 1H, H5): 6.5 - 6.8 (Qt, 2H, H3+H4): 2.9 - 3.1(m, 6H, $-CH_2-$): 2.5 (m, 2H, $-CH_2-$): 1.3 - 1.7 ( m, 8H, $-CH_2-$)

Analysis: $C_{32}H_{46}N_4S_2O_6$ requires: C: 59.41, H: 7.17, N: 8.66, S: 10.09. Found: C: 60.24, H: 7.15, N: 8.44, S: 8.67.

(2) N,N-(6,6)-[(3,3-dithiobispropionyl)-N-(6-aminohexyl)anthranilic acid]-bis-(2-chloro-4-amidino)phenyl ester hydrochloride.

The above compound (97 mg) was mixed with anhydrous p-toluenesulphonic acid (10 mg) and 2-chloro 4-amidinophenol HCl (93 mg, 3.0 mol equivalent) in dry pyridine (2.0 ml) and N,N dicyclohexylcarbodiimide (77 mg, 2.5 mol equivalent) added. After stirring for 48h at ambient temperature, the solution was filtered and evaporated to dryness. The yellow oil was recrystallised from $EtOH/H_2O$ 1:5 v/v (5.0 ml) with addition of saturated brine (2.0 ml) and then from the same solvent without brine. The deposited solid was dried _in vacuo_. Yield 76.7 mg. Glassy solid m.p. $145^oC$.

'H Nmr. (DMSO $d^6$) δ: 9.45 (broad S, 4H Exch. $D_2O$, Amidine H): 8.15 (d, 1H, amidinophenyl H3): 8.05 (dd, 1H, amidinophenyl H5): 7.96 (irregular triplet, 1H shifted in $D_2O$, NH): 7.89 (irr. d, 1H, amidinophenyl H6): 7.72 (dd, 1H anthraniloyl H6): 7.50 (irr. 1H, anthraniloyl H5): 7.40 (irr. t, 1H, exch. $D_2O$, NH): 6.85 (d, 1H, anthraniloyl H3): 6.71 (t, 1H, anthraniloyl H4): 3.20 (broad d, 2H, $-CH_2NHCO-$): 3.00 (irr. d, 2H, $-CH_2NH$ anthraniloyl): 2.86 (t,2H, $-COCH_2-$): 2.4 (t, 2H, $-COCH_2CH_2S-$): 1.50 (m, 2H, $-CH_2-$): 1.2 - 1.4 (m, 6H, $-CH_2-$).

The bis-(4-amidinophenyl) ester of compound (1) was prepared in a similar fashion. Both compounds formed dimers of plasmin as measured by HPLC under the conditions described in Example 6 above.

In the figures:

Figure 1: SDS PAGE/fibrin zymography characterisation of the conjugate of Example 7.

Figure 2: SDS PAGE/fibrin zymography characterisation of the conjugate of Example 8.

In both figures:

Conditions: 8% P.A.G.E., fibrin overlay zymography.Activities in SU/ml.

H = hydrolysed at 37$^{o}$C for 2h in TGS buffer.

Claims C

1. A conjugate comprising a plurality of fibrinolytic enzymes linked together through the active centres thereof by means of a removable blocking group.

2. A conjugate according to claim 1, comprising two fibinolytic enzymes linked together as defined.

3. A conjugate according to claim 2, wherein each enzyme is independently human plasmin or human tissue plasminogen activator.

4. A conjugate according to any preceding claim, wherein the removable blocking group has the formula (I):

$$L(-B-A-X-)n \qquad (I)$$

wherein:

n is an integer of 2 to 6;

L is a nodal group of valency n;

each B is independently a linear hydrophilic linking group;

each A is independently a bridging group comprising at least one heteroatom selected from oxygen, sulphur and nitrogen in which the nitrogen may be optionally substituted by $C_{1-6}$ alkyl; and

each X is independently an acyl group of formula

$- R - \overset{O}{\overset{\|}{C}} -$ in which R is an aromatic or unsaturated non-aromatic function.

5. A conjugate according to claim 4 in which n is 2 and the moiety $L(-B-)_2$ is a linear hydrophilic polymer.

6. A conjugate according to claim 4 or 5 in which the moiety A is of formula (II):

$$-(C)_m-D-E- \qquad (II)$$

wherein C is a functional group suitable for attachment to B;

D is a chemical bond or a hydrocarbon chain;

E is an electron releasing group; and

m is 1, or when E is suitable for attachment to B, m may instead be O.

7. A conjugate according to any one of claims 4 to 6 in which the moiety X is optionally substituted benzoyl or acryloyl, further substituted by the group A.

8. A conjugate according to claim 1, in which the blocking group is derived from α, -O,O'-bis-(4-carboxyphenyl)polyethylene glycol,

α,ω-O,O'-bis- [N-2-carbonyl(4'-hydrazinobenzoic acid)]polyethyleneglycol,

α,ω-O,O'-bis- [N-6-(N'-carbonylamino)hexylanthranilic acid] polyethylene glycol,

α,ω-O,O'-bis-(N-[2-(N'-carbonylaminoethyl)-4'-amino] benzoic acid) polyethylene glycol or

N,N-(6,6)-[(3,3-dithiobispropionyl)-N-(6-aminohexyl)-anthranilic acid].

9. α,ω-O,O'-bis-(4-carboxyphenyl)polyethylene glycol 400, bis-[human lys77-(ser 740-yl)plasmin]ester,

α-[human(ser 478-yl)tissue plasminogen activator],ω-[human lys77-(ser 740-yl)plasmin], O,O'-bis-(N-2-carbonyl- [4'-hydrazinobenzoic acid])polyethylene glycol 600,

α-[human(ser 478-yl)tissue plasminogen activator],ω-[human lys77-(ser 740-yl)plasmin], O,O'-bis-(N-[2-(N'- carbonylaminoethyl)-4'-amino]benzoic acid)polyethylene glycol 600,

α,ω-bis- (human [ser 478-yl]-tissue plasminogen activator), O,O'-bis-(N-2-carbonyl-[4'-hydrazinobenzoic acid]) polythene glycol 600,

bis-α,ω-(human [ser 478-yl] tissue plasminogen activator), O,O'-bis-(N-2-carbonyl-[4'-hydrazinobenzoic acid])polyethylene glycol 1500,

α-[human (ser 478-yl) tissue plasminogen activator],ω-[human lys77-(ser 740-yl) plasmin],O,O'-bis-(N-2-carbonyl-[4'-hydrazinobenzoic acid])polyethylene glycol 1500,

α-[human (ser 478-yl) tissue plasminogen activator],
ω-[human lys77-(ser 740-yl)plasmin],
O,O′-bis-(N-6-[$N^{\varepsilon}$-carbonylamino]hexylanthranilic acid) polyethylene glycol 600 or

α,ω-N,N-(6,6)-[(3,3-dithiobispropionyl)-N-(6-aminohexyl)-anthranilic acid] -bis-[human lys77-(ser 740-yl)plasmin] ester.

10. A process for preparing an enzyme conjugate according to claim 1, which comprises reacting one or more fibrinolytic enzymes simultaneously or sequentially with a polyfunctional acylating agent.

11. A process for preparing an enzyme conjugate according to claim 1, which comprises reacting together one or more enzymes whose active centres are blocked by groups capable of reacting together to form a reversible blocking group.

12. A pharmaceutical composition comprising an enzyme conjugate according to any of claims 1 to 9 in combination with a pharmaceutically acceptable carrier.

13. A compound of formula (III):

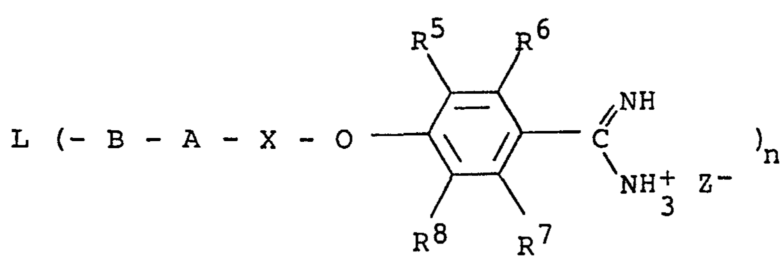

(III)

where L,B,A,X and n are as defined in claim 4; $R^5$,$R^6$,$R^7$ and $R^8$ are each independently hydrogen or an electron withdrawing group; and Z is a counter-anion.

14. α,ω-O,O'-bis-(4-carboxyphenyl)polyethylene glycol 400, bis-4-amidinophenyl ester p-toluenesulphonate,

α,ω-O,O'-bis-(4-carboxyphenyl)polyethylene glycol 1500, bis-4-amidinophenyl ester p-toluenesulphonate,

α,ω-O,O'-bis-[N-2-carbonyl(4'-hydrazinobenzoic acid)] polyethylene glycol 600, bis-4-amidinophenyl ester hydrochloride,

α,ω-O,O'-bis-[N-6-(N'-carbonylamino)hexyl- anthranilic acid]polyethylene glycol 600, bis-4- amidinophenyl ester hydrochloride,

α,ω-O,O'-bis-(N-[2-(N'-carbonylaminoethyl)-4'- amino] benzoic acid)polyethylene glycol 600, bis-4-amidinophenyl ester hydrochloride,

α,ω-O,O'-bis-(N-[2-(N'-carbonylaminoethyl)-4'-amino]benzoic acid)polyethylene glycol 600, bis-2-chloro-4-amidinophenyl ester hydrochloride,

α,ω -O,O′-bis - [N-2-carbonyl-(4'-hydrazinobenzoic acid)]polyethylene glycol 1500, bis-4-amidinophenyl ester hydrochloride or

N,N-(6,6)-[(3,3-dithiobispropionyl)-N-(6-amino hexyl)-anthranilic acid]-bis-(2-chloro-4-amidino) phenyl ester hydrochloride.

15. A conjugate according to any of claims 1 to 9, for use as an active therapeutic substance.

16 A conjugate according to any of claims 1 to 9, for use in the treatment of thrombotic diseases.

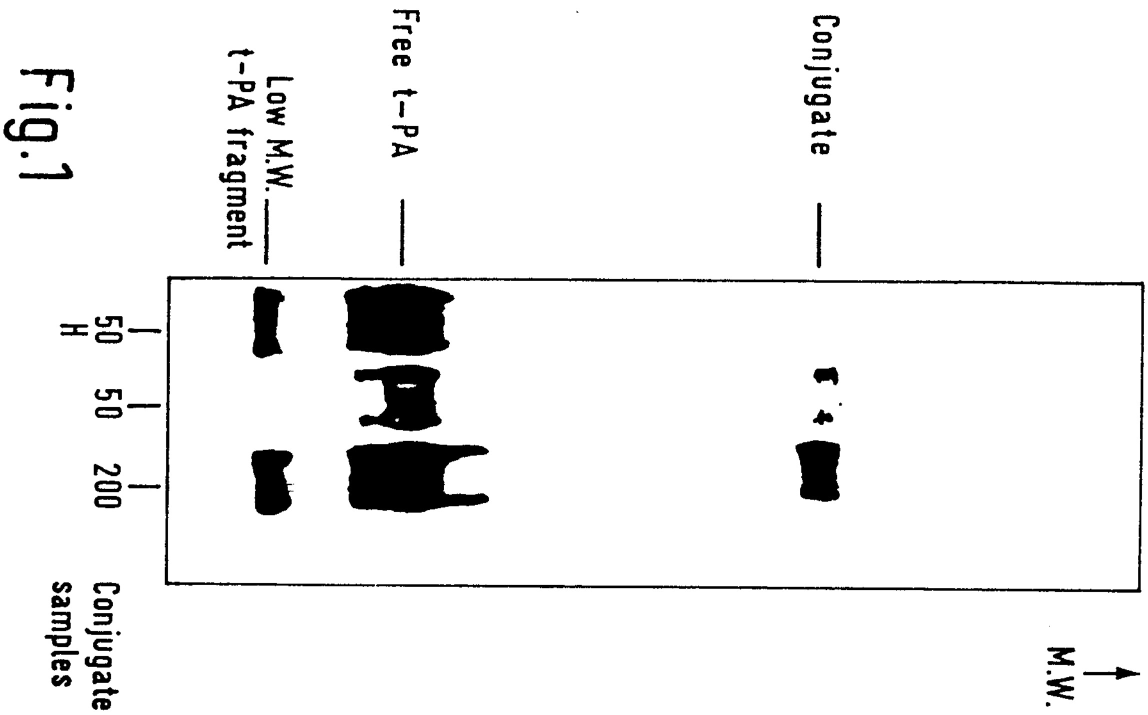
Conjugate
Free t-PA
Low M.W. t-PA fragment
M.W.
50 H
50
200
Conjugate samples

Fig.1

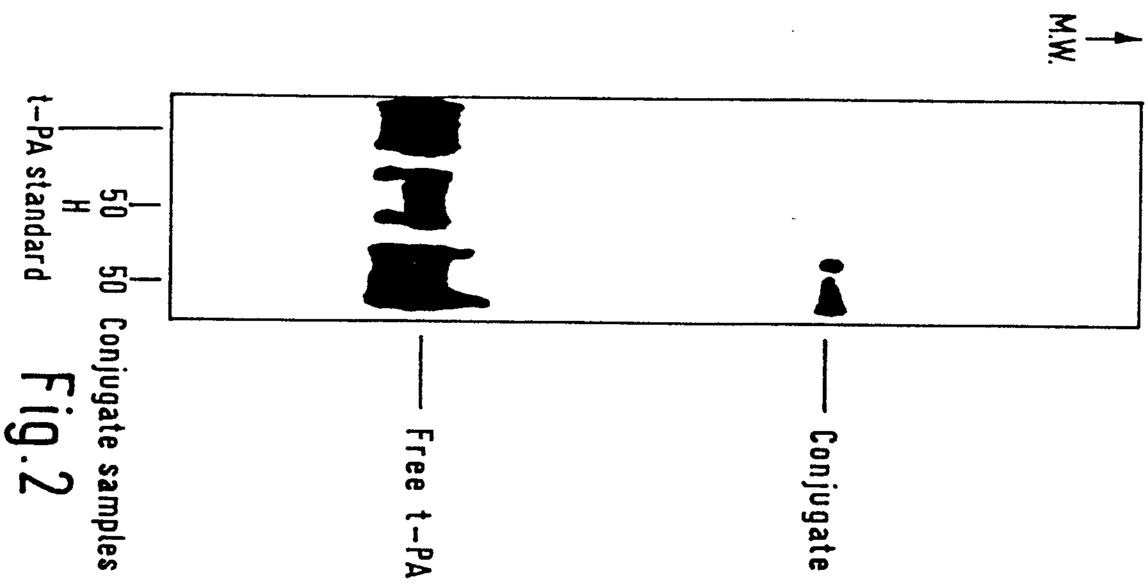
M.W.
t-PA standard
50 H
50
Conjugate samples
Free t-PA
Conjugate

Fig.2